# EUROPEAN PATENT APPLICATION

(11) **EP 4 350 007 A1**
(43) Date of publication of application: **10.04.2024**
(21) Application number: 22810288.5
(22) Date of filing: 26.04.2022
(51) Int. Cl.: C12Q 1/6874, C12N 15/11

(54) **SEQUENCING METHOD**

(30) Priority: 24.05.2021 CN 202110566022
(71) Applicant: GeneMind Biosciences Company Limited, Shenzhen, Guangdong 518023 (CN)
(72) Inventor: LIU, Lei, Shenzhen, Guangdong 518023 (CN); LUO, Weiwei, Shenzhen, Guangdong 518023 (CN); FAN, Jicai, Shenzhen, Guangdong 518023 (CN); LU, Yongyi, Shenzhen, Guangdong 518023 (CN); CHEN, Fang, Shenzhen, Guangdong 518023 (CN); SUN, Lei, Shenzhen, Guangdong 518023 (CN)
(74) Representative: Schiweck Weinzierl Koch Patentanwälte Partnerschaft mbB
(86) International application number: PCT/CN2022/089147
(87) International publication number: WO 2022/247555

(57) **Abstract**

Disclosed is a sequencing method, the method comprising providing a solid phase substrate that has a plurality of single-stranded nucleic acids attached to a surface thereof, wherein the 5' ends of the single-stranded nucleic acids are attached to the surface, the single-stranded nucleic acids are polynucleotides that contain an insert-first sequence, the insert is a nucleic acid sequence from a sample to be tested, the first sequence is a preset sequence containing an index-first site, the index is a preset sequence that specifically corresponds to the sample to be tested; providing a first sequencing primer, wherein the sequencing primer can hybridize with the 5' end of the first site; hybridizing the first sequencing primer with the single-stranded nucleic acids and placing same under a condition suitable for polymerized sequencing, so as to determine part of the sequence of the single-stranded nucleic acids by extending the first sequencing primer, so as to obtain a sequencing result. Said multiplex sequencing method can effectively reduce the level of index hopping, and is particularly suitable for a situation in which trace species or rare variants in a mixed sample need to be accurately detected.

## Description

### TECHNICAL FIELD

The present disclosure relates to the field of nucleic acid detection, particularly to the field of sequencing, and more particularly to a method suitable for sequencing a tag library, a kit, and a system.

### BACKGROUND

Next-generation sequencing, also referred to as high-throughput sequencing or massively parallel sequencing, enables the determination of nucleic acid sequences of multiple samples in one sequencing run. One way to achieve this determination is multiplex sample analysis, also commonly referred to as multiplex library or multiplex sequencing.

Multiplex sequencing adds to each DNA fragment a specific sequence uniquely corresponding to a sample from which a DNA fragment is derived in the library construction process, such that a library of multiple samples can be mixed in one reaction system for sequencing to acquire sequencing data, and the sequencing data can be distributed to corresponding samples according to the specific sequence, thereby acquiring the sequencing data of each sample, where the specific sequence is usually referred to as a tag, an index, or a barcode.

An error in tag assignment among the multiplex libraries, also known as index hopping (or, index misassignment or sample cross-talk), is a known problem for multiplex sequencing.

This was found by Kircher et al., who proposed a solution. They designed a double-indexing test in which tags were introduced into the adapters at the two ends of the library to quantitatively detect the index hopping level, and found that in multiplex sequencing, the tag misassignment rate was about 0.3%, several orders of magnitude higher than expected. Also, Kircher et al., further disclosed that the double-indexing method identifies a sample by double-tag cross validation at the two ends, and can exponentially decrease the tag misassignment rate and significantly reduce the index hopping level (Kircher et al., 2012, Nucleic Acids Res., Vol. 40, No. 1).

Later, with the development of high-throughput sequencing technology, especially with the adoption of a sequencing platform for amplifying a nucleic acid under test by using an exclusion amplification (ExAmp) technique on a patterned flow cell to give a molecular cluster, the index hopping problem has become apparent. Therefore, Illumina proposed a double-indexing library strategy. UDIs, or unique dual indexes are added to the P5 and P7 ends of the library, and by the P5 Index 2/P7 Index 1 pairing design and cross validation of indexes at the two ends, the index hopping problem revealed in such sequencing platforms is resolved (Illumina, 2017, Effects of Index Misassignment on Multiplexing and Downstream Analysis White Paper).

It will be appreciated that, assays that involve the use of high-throughput sequencing to seek for trace "positive" data in a mixture with high background noise interference are very susceptible to index hopping, including cancer genomics and other applications requiring precise detection of rare variations, such as liquid biopsy, etc. With the development and advancement of sequencing platforms and sequencing applications, it is necessary to further reduce index hopping or to provide alternative methods that can reduce index hopping.

### SUMMARY

Embodiments of the present disclosure are intended to at least solve, to some extent, one of the technical problems existing in the prior art or at least provide a useful alternative. Accordingly, embodiments of the present disclosure provide a sequencing method.

It should be noted that the sequencing method of the present disclosure is based on the following summary and findings:
Theoretically and generally, errors may be present during the preparation of a library, the immobilization or attachment of a library to the surface of a solid carrier, or the amplification of nucleic acid molecules on the surface of a solid carrier, leading to index hopping, but the specific mechanism of occurrence is unclear.

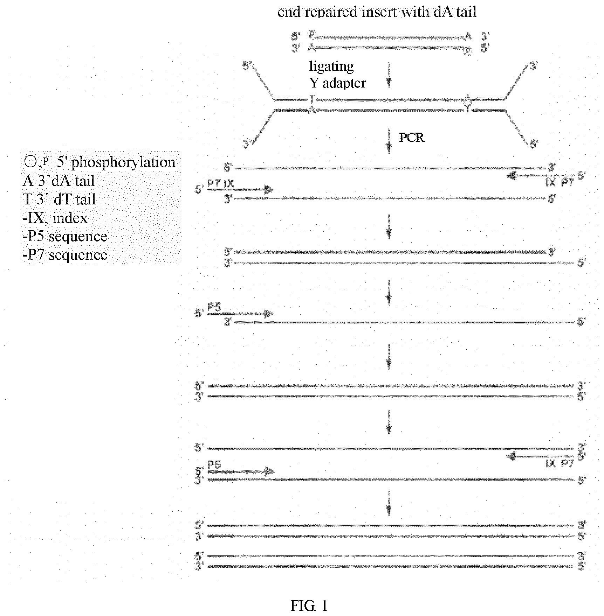

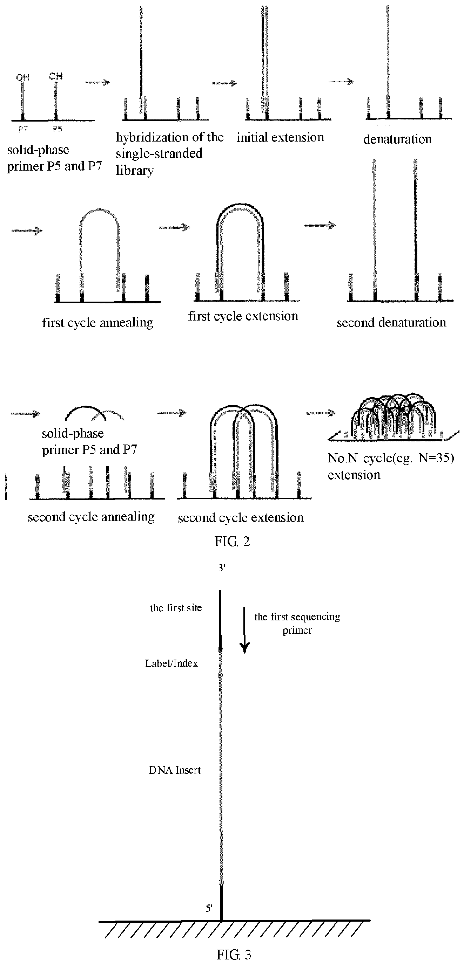

By setting and configuring the samples, and utilizing a mainstream sequencing platform such as an Illumina high-throughput sequencing platform according to the manual instruction, the inventor designed study (a): Single-tag libraries are separately constructed based on multiple samples, such that the libraries of different samples include different tags (the samples correspond to the tags respectively). The construction of single-tag libraries, as shown in FIG. 1, includes: (i) conducting end-repairing on the target sequence (fragment under test/insert) and adding A; (ii) ligating Y adapters to the two ends of the target sequence from step (i) by TA sticky end ligation; (iii) synthesizing a first nascent strand including a tag at a 5' end by extending a primer (P7 primer, including P7 sequence) capable of hybridizing with a 3' end of the ligation product from step (ii) and including a tag; (iv) synthesizing a second nascent strand by extending a primer (P5 primer, including P5 sequence) capable of hybridizing with a 3' end of the first nascent strand; and (v) amplifying the second nascent strand using the P7 primer and the P5 primer to give a single-tag library where the tag is located at the side of the 3' end of the target sequence (also referred to as P7 end). The single-tag libraries corresponding to different samples are mixed to give a mixture library, which is then loaded on the surface of a chip for amplification. For example, as shown in FIG. 2, a bridge amplification is conducted on the surface of the substrate, or specifically, the mixture library is melted to give a single-stranded library. The single-stranded library is hybridized with a substrate having two primers/probes immobilized on the surface thereof, which may be referred to as a P7 solid-phase primer and a P5 solid-phase primer and respectively hybridizable with 3' ends of the two complementary strands of the library, corresponding to the above library construction. The P7/P5 solid-phase primers are extended to synthesize the complementary strands of the single-stranded library. New single-stranded templates (complementary strands) are acquired by denaturation, annealing is conducted to allow the complementary strands to hybridize with the P5/P7 primers, and the P5/P7 primers are extended to synthesize new complementary strands. As such, by one or more runs of denaturation - annealing - extension, an amplification product (clonal cluster) is acquired. Single-end sequencing or double-end sequencing is then conducted on the amplification product to give a sequencing result A.

The inventor also designed study (b): Double-indexing libraries are constructed on the same samples, where the construction of the double-indexing libraries is similar to that in FIG. 1. In step (iv), a second nascent strand is synthesized by using a P5 primer including a second tag, such that the P7 end of the second nascent strand includes a first tag while the P5 end includes a second tag. Accordingly, two tags are introduced and respectively located at the side where the 3' end of the fragment under test is located (or P7 end) and the side where the 5' end of the fragment under test is located (or P5 end), so as to acquire the double-indexing library. Also, similar to study (a), the double-tag libraries corresponding to different samples are mixed to give a mixture library, which is then loaded on the surface of a chip to conduct the same amplification and sequencing as in study (a) to give a sequencing result B, where the library construction and sequencing of study (b) are the same as the double-tag library detection reported by Kircher et al (the tags at the ends P7 and P5 are respectively read by taking the forward strand and the reverse strand as templates).

In addition, based on the same sample, the inventors designed studies (c) and (d). In study (c), a single-tag library with a tag on the side of the 5' end of the target sequence (P5 end) (P7 primer includes no tag and P5 primer includes a tag) is constructed according to the preparation process of the single-tag library with a tag on the side of the 3' end of the target sequence (P7 end) in the above study (a), and the mixing and solid-phase amplification of the single-tag library are the same as in study (a). In addition, according to the P5 solid-phase primer or the P5 end sequence design, a primer capable of hybridizing with the 3' end of the reverse strand of the library is synthesized as a sequencing primer that can be used freely for the determination of the P5 end tag, so as to give a sequencing result C. In study (d), double-tag libraries are prepared according to the method for preparing double-tag libraries in study (b) above, and the mixing and solid-phase amplification of the double-tag library are the same as in study (a). In addition, as in study (c), according to the P5 solid-phase primer or the P5 end sequence design, a primer capable of hybridizing with the 3' end of the reverse strand of the library is synthesized as a sequencing primer that can be used freely for the determination of the P5 end tag to read the two tags and at least a part of the target sequence on the same single-stranded template, so as to give a sequencing result D.

The above studies (a), (b), (c), and (d) correspond to the same sample, and the sequencing data is processed using the same demultiplex/demultiplexing method, including assigning the sequencing data to the corresponding sample according to the sequence information of the tag or tag set (dual tags), to give corresponding sequencing results A, B, C, and D.

However, the inventor surprisingly found that, for the same single-tag library sequencing, the index hopping level in sequencing result C was significantly lower than that in sequencing result A, by about 1/10,000. In other words, the index hopping level in sequencing result C was comparable to the index hopping level in the tested double-indexing sequencing result B (as reported by Kircher et al.). For the double-tag library, generally, as reported, the index hopping level of a double-tag library is significantly lower than that of a single-tag library, by about 1/100,000. As seen from the data of the mixture sample of microorganisms of these studies, the sequencing result D had an index hopping level slightly lower than the ratios disclosed by Kircher et al. Unaccountably, it seems that at/near which end of the fragment under test or at which position in the single-stranded nucleic acid template the tag is located, the order in which the tag(s) is/are introduced into the library template, and/or whether the tag is located at the end of the single-stranded nucleic acid template proximal or distal to the surface, may affect the occurrence of index hopping. Alternatively, to some extent, the plurality of nucleic acid molecules included in the tag library constructed according to the above method appear to be composed of two sequences, a forward strand and a reverse strand, that are completely complementary and identical/symmetrical in information. Theoretically, reading the same or complementary parts of either or both of the two sequences may finally give the same sequencing result. However, inexplicably, in terms of the frequency of index hopping, the reading results of the complementary parts of the two sequences are inconsistent/not completely symmetrical or significantly different.

Based on this finding, an embodiment of the present disclosure provides a sequencing method, including: providing a solid substrate having a surface connected with a plurality of single-stranded nucleic acids, where 5' ends of the single-stranded nucleic acids are connected to the surface, the single-stranded nucleic acids are polynucleotides including an insert (or insert fragment) - a first sequence, the insert is a nucleic acid sequence from a sample under test, the first sequence is a predetermined sequence including a tag - a first site, and the tag is a predetermined sequence with specificity to the sample under test; providing a first sequencing primer capable of hybridizing with a 5' end of the first site; and hybridizing the first sequencing primer with the single-stranded nucleic acid and placing under a condition suitable for polymerization sequencing to determine a part of the sequence of the single-stranded nucleic acid by extending the first sequencing primer, so as to acquire a sequencing result.

An embodiment of the present disclosure further provides a system for implementing the sequencing method, which is an automatic device for implementing the sequencing method, including: a mechanical mechanism for holding the solid substrate; a liquid path structure connected with the mechanical mechanism for introducing a first sequencing primer, DNA polymerase and the like into the solid substrate, including a pump; and a control unit connected with the mechanical mechanism and the liquid path structure for enabling the hybridization and/or enabling the presence of substances on the solid substrate in an environment suitable for polymerization sequencing.

An embodiment of the present disclosure further provides a kit for implementing the sequencing method according to the above embodiment, including the solid substrate and the first sequencing primer.

An embodiment of the present disclosure further provides a computer product, including a memory for storing a program and a control system, where the control system executes the program to implement the sequencing method according to the above embodiment.

The above method, system, and/or computer product are based on the above surprising findings. Though unaccountable, the method or the system for implementing the method can reduce the frequency of index hopping to 1/10,000 by locating a single tag at a designated position on a single-stranded nucleic acid template and determining the tag and at least a part of a fragment under test (insert) from a sample, etc., in the template, and are suitable for sequencing tagged mixture libraries/samples, particularly determination of mixture samples sensitive to index hopping, for example, cancer genomics and other applications requiring precise detection of rare variations such as liquid biopsy, the field of pathogen detection such as low copy pathogen or bacterial species detection in metagenomic samples, etc.

Additional aspects and advantages of the embodiments of the present disclosure will be partially set forth in the following description, and will partially become apparent from the following description or be appreciated by practice of the embodiments of the present disclosure.

### BRIEF DESCRIPTION OF THE DRAWINGS

The above and/or additional aspects and advantages of the embodiments of the present disclosure will become apparent and easily understood from the description of the embodiments with reference to the following drawings, among which:
FIG. 1 is a schematic diagram of single-tag library construction using incomplete adapters adaptive to a commercially available mainstream sequencing platform (Illumina);
FIG. 2 is a schematic diagram of acquiring clonal clusters on the surface of a solid substrate adaptive to a commercially available mainstream sequencing platform (Illumina);
FIG. 3 is a schematic diagram of a sequencing method according to an embodiment of the present disclosure;
FIG. 4 is a schematic diagram of a sequencing method according to an embodiment of the present disclosure;
FIG. 5 is a schematic diagram of a sequencing method according to an embodiment of the present disclosure;
FIG. 6 is a schematic diagram of a sequencing method according to an embodiment of the present disclosure;
FIG. 7 is a schematic diagram of a sequencing method according to an embodiment of the present disclosure;
FIG. 8 is a schematic diagram of an incomplete adapter structure and an amplification scheme according to an embodiment of the present disclosure;
FIG. 9 is a schematic diagram of an intact adapter structure and an amplification scheme according to an embodiment of the present disclosure; and
FIG. 10 is a schematic diagram of a library structure on the surface of a chip according to an embodiment of the present disclosure.

### DETAILED DESCRIPTION

The embodiments of the present disclosure are described in detail below, and the examples of the embodiments are shown in the accompanying drawings, throughout which identical or similar reference numerals represent identical or similar elements or elements having identical or similar functions. Reference numerals and/or letters may be repeatedly used in different examples in the present disclosure for simplicity and clarity rather than for indicating the relationship between various embodiments and/or settings discussed. The embodiments described below by reference to the accompanying drawings are exemplary and illustrative, and should not be construed as limiting the present disclosure.

As used herein, the singular forms "a", "an", "the", and the like, include plural referents unless otherwise indicated; "a set of" or "a plurality of" refers to two or more.

As used herein, unless otherwise indicated, the terms "first", "second", "third", "fourth", and the like are used for illustrative purposes only, and should not be construed as indicating or implying the relative importance or implicitly indicating the number of indicated technical features; a feature defined by "first", "second", and the like may explicitly or implicitly include one or more of the features.

As used herein, unless otherwise indicated, the term "nucleotide" refers to four natural nucleotides (e.g., dATP, dCTP, dGTP and dTTP, or ATP, CTP, GTP and UTP) or derivatives thereof, and is sometimes directly referred to as the base included (A, T/U, C, G). The reference to a nucleotide or base in a particular embodiment may be known to those of ordinary skills in the art in light of the context.

As used herein, unless otherwise indicated, single-stranded or double-stranded nucleic acid molecules, including the inserts, nucleic acid fragments, sequences, sites, polynucleotides, adapters, primers/probes, etc., are written in a 5'-to-3' direction from left to right.

As used herein, unless otherwise indicated, "connect", "ligate", "immobilize", and the like are to be construed in their broader sense, for example, as being capable of being connected fixedly, reversibly, directly, indirectly via an intermediate, via a chemical bond (e.g., a covalent bond), or by chemical or physical adsorption, etc.

As used herein, an adapter, a primer, or a probe, is an oligonucleotide fragment with a predetermined or known sequence. The adapter is a single-stranded or double-stranded nucleic acid molecule, while the primer or the probe is a single-stranded oligonucleotide. In commercially available mainstream sequencing platforms, the end of a nucleic acid fragment under test (also referred to as an insert) from a sample is generally provided with a predetermined sequence (adapter) by processing, and the fragment under test is connected or immobilized to a designated position of a reactor (such as a flow cell or a designated surface of a chip) by using a primer or a probe (oligonucleotide strand) complementary to or binding to at least a part of the adapter. Based on the base complementary principle, at least a part of the sequence of the adapter can be used to design a primer/probe, and can be used as a binding site for a specific primer/probe.

As used herein, the term "sequencing" refers to sequence determination, and is used interchangeably with "nucleic acid sequencing" and "gene sequencing" to refer to the determination of base order in nucleic acid sequences, including sequencing by synthesis (SBS) and/or sequencing by ligation (SBL), including DNA sequencing and/or RNA sequencing, including long fragment sequencing and/or short fragment sequencing (the long fragment and short fragment are defined relatively; for example, nucleic acid molecules longer than 1 Kb, 2 Kb, 5 Kb or 10 Kb may be referred to as long fragments, and nucleic acid molecules shorter than 1 Kb or 800 bp may be referred to as short fragments), and including double-end sequencing, single-end sequencing, paired-end sequencing, and/or the like (the double-end sequencing or paired-end sequencing may refer to the reading of any two segments or portions of the same nucleic acid molecule that are not completely overlapping).

The sequencing includes the process of binding nucleotides (including nucleotide analogs) to a template and acquiring the corresponding reaction signals. Some sequencing platforms where the binding of nucleotides to the template and the acquisition of reaction signals are conducted asynchronously/in real-time generally involve multiple cycles of sequencing to determine the order of multiple nucleotides/bases on the template. A "cycle of sequencing", also referred to as "sequencing cycle", may be defined as one base extension of the four nucleotides/bases, and in other words, as the determination process of the base type at any given position on the template. For sequencing platforms that achieve sequencing based on polymerization or ligation reactions, one cycle of sequencing includes the process of binding four nucleotides to the template at a time and acquiring the corresponding reaction signals. For platforms that achieve sequencing based on polymerization reaction, a reaction system includes reaction substrate nucleotides, a polymerase, and a template; a predetermined sequence (a sequencing primer) is bound to the template, and on the basis of the base pairing principle and the rationale of polymerization reaction, the added reaction substrate (nucleotides) is controllably connected to the 3' end of the sequencing primer under the catalysis of the polymerase to achieve the pairing with the base at a corresponding position of the template. Generally, one cycle of sequencing may include one or more base extensions (repeats). For example, four nucleotides are sequentially added to the reaction system to each perform base extension and corresponding acquisition of reaction signals, and one cycle of sequencing includes four base extensions; for another example, four nucleotides are added into the reaction system in any combinations (such as in pairs or in one-three combinations), the two combinations each perform base extension and corresponding acquisition of reaction signals, and one cycle of sequencing includes two base extensions; for yet another example, four nucleotides are added simultaneously to the reaction system for base extension and reaction signal acquisition, and one cycle of sequencing includes one base extension. Sequencing can be performed through a sequencing platform, which may be selected from, but is not limited to, the Hiseq/Miseq/Nextseq/Novaseq sequencing platform (Illumina), the Ion Torrent platform (Thermo Fisher/Life Technologies), the BGISEQ and MGISEQ/DNBSEQ platforms (BGI) and single-molecule sequencing platforms. The sequencing method may be selected from single-read sequencing and double-end sequencing. The acquired sequencing results/data (i.e., read fragments) are referred to as reads, and the length of a read is referred to as read length.

As used herein, the term "solid substrate" may be any solid support useful for immobilizing nucleic acid sequences, such as nylon membranes, glass slides, plastics, silicon wafers, magnetic beads, and the like, and may sometimes be referred to as a reactor, chip, or flow cell.

According to an embodiment of the present disclosure, as shown in FIG. 3, a sequencing method is provided, including: providing a solid substrate having a surface connected with a plurality of single-stranded nucleic acids, where 5' ends of the single-stranded nucleic acids are connected to the surface, the single-stranded nucleic acids are polynucleotides including an insert - a first sequence, the insert is a nucleic acid sequence from a sample under test, the first sequence is a predetermined sequence including a tag - a first site, and the tag is a predetermined sequence with specificity to the sample under test; providing a first sequencing primer capable of hybridizing with a 5' end of the first site; and hybridizing the first sequencing primer with the single-stranded nucleic acid and placing under a condition suitable for polymerization sequencing to determine a part of the sequence of the single-stranded nucleic acid by extending the first sequencing primer, so as to acquire a sequencing result.

The method is disclosed on the basis of the foregoing surprising findings. Though unaccountable, the method can reduce the frequency of index hopping to 1/10,000 by locating a single tag at a designated position on a single-stranded nucleic acid template, spacing from the surface a certain distance, and determining the tag and at least a part of a nucleic acid sequence (insert) from a sample, etc., in the template, and are suitable for sequencing tagged mixture libraries/samples, particularly determination of mixture samples sensitive to index hopping. In specific, the method is particularly useful in detection applications that seek for trace "positive" data in a mixture with high background noise, such as cancer genomic applications requiring precise detection of rare variations, the field of pathogen detection such as low copy pathogen or bacterial species detection in microorganism samples, etc.

The insert (or DNA insert) is a nucleic acid sequence from the sample, which is the sequence unknown/under test in a template under test (single-stranded nucleic acid). The first sequencing primer may be free/non-immobilized, e.g., in a solution, or may be a solid-phase primer, e.g., having a 5' end connected with the surface of a solid substrate. In a certain specific example, the first sequencing primer is in a free state.

In a certain example, the tag is directly ligated to the insert (no nucleotides/bases therebetween), the reads acquired by extending the first sequencing primer include the determined sequence information of the tag and the sequence information of at least a part of the insert, and the subsequent demultiplexing can acquire the sequence information of the tag in the reads based on the length of the tag, so as to assign data to the corresponding samples.

The sequencing result includes a plurality of reads. In a certain specific example, the length of the read is not less than four times the length of the tag, and the length of the determined insert excluding the tag sequence information for indicating the sample in the read is not less than three times the tag length. Preferably, the length of the read is not less than five, six, seven, eight, ten, or fifteen times the length of the tag, and the like, and in the case that the accuracy of the generated data meets the predetermined requirement, a longer read length and/or a higher throughput may facilitate the development of more application tests or may meet the requirements of more application tests.

It will be appreciated that the reading tags will take up a part of the read length. Thus the length of the tag is usually set as 6-12 nt, such that the tags are sufficiently short but can effectively distinguish a certain number of samples after the tags are mixed. For short fragment sequencing, tags of 6 nt or 8 nt are commonly used, so as to provide a sufficient number of tags available for mixing to allow the determination of a certain number of samples in one sequencing run and the acquisition of sequence information from nucleic acids as long as possible or samples as many as possible.

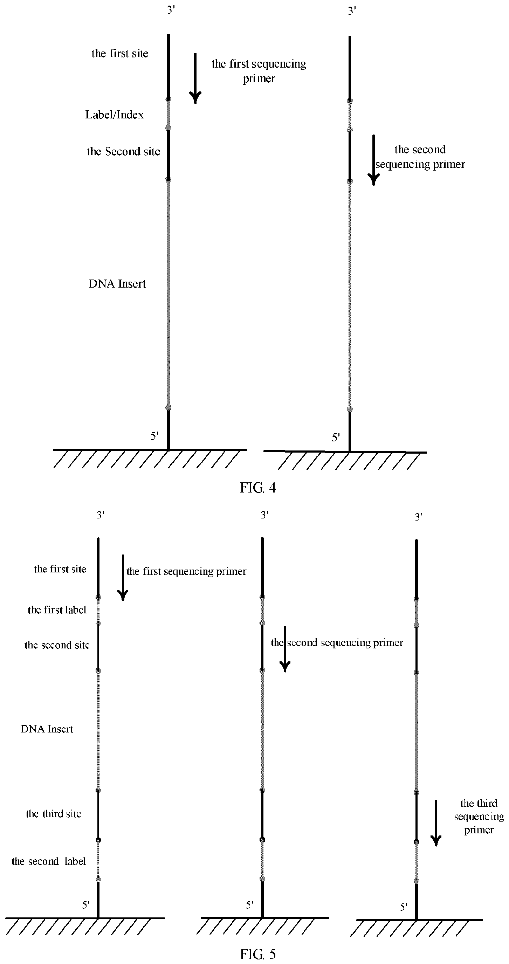

In some certain examples, referring to FIG. 4, the first sequence is a predetermined sequence including a second site - the tag - the first site, and the method further includes: providing a second sequencing primer capable of hybridizing with a 5' end of the second site; and hybridizing the second sequencing primer with the single-stranded nucleic acid and placing under a condition suitable for polymerization sequencing to determine at least a part of the sequence of the insert on the single-stranded nucleic acid by extending the second sequencing primer, so as to acquire the sequencing result.

By designing and jointly using the first sequencing primer and the second sequencing primer, the method is favorable for quickly acquiring the sequencing result, due to the capability of detecting at least a part of the tag sequence and the insert without synthesizing a new chain or changing a template. Specifically, the first sequencing primer and the second sequencing primer are both free primers. The obtained sequencing result includes a first read and a second read. The first read includes sequence information of the tag, and the second read includes sequence information of at least a part of the insert. As such, subsequent demultiplexing (or spliting) and distribution of sequencing data are facilitated.

It will be appreciated that the order of the procedures, for example, whether the first sequencing primer or the second sequencing primer is first used for sequencing, whether the first sequencing primer or the second sequencing primer is first provided or the first sequencing primer and the second sequencing primer are simultaneously provided, or the like, does not affect the acquisition of the corresponding sequencing result, and is thus not specified in the method. The sequencing methods in the following examples are similar to those above, and those skilled in the art will be appreciated whether the acquisition of the corresponding sequencing result in the relevant examples requires the sequence of executing the relevant procedures, unless otherwise stated.

In some certain examples, the single-stranded nucleic acid is a polynucleotide including a second sequence - the insert - the first sequence, the second sequence is a predetermined sequence including a third site, and the single-stranded nucleic acid is covalently attached to the surface of the solid substrate via a 5' end of the second sequence. In a certain specific example, the template (single-stranded nucleic acid) is prepared by ligating an adapter to the end of the insert, the second site and the third site are introduced by ligation with the same adapter, and the second site and the third site are reverse complementary sequences.

In some certain examples, the tag is a first tag, the second sequence is a predetermined sequence including a second tag - the third site or a predetermined sequence including a fourth site - the second tag - the third site, and the second tag is a predetermined sequence with specificity to the sample under test. The second tag is a predetermined fragment with a sequence different from that of the first tag. Combined use of double/multiple tags and cross validation using the tags will facilitate a more accurate demultiplexing of mixed sequencing data to corresponding samples.

Specifically, in a certain example, referring to FIG. 5, the method further includes: providing a third sequencing primer capable of hybridizing with a 5' end of the third site; and hybridizing the third sequencing primer with the single-stranded nucleic acid and placing under a condition suitable for polymerization sequencing to determine the sequence of the second tag on the single-stranded nucleic acid by extending the third sequencing primer, so as to acquire the sequencing result. It will be appreciated that the sequencing result further includes a third read including sequence information of the second tag. The order of executing the procedures of extending the first sequencing primer, the second sequencing primer, or the third sequencing primer to determine the corresponding sequences is not specified in the embodiment.

By designing and jointly using the first sequencing primer, the second sequencing primer, and the third sequencing primer to determine the three parts (the insert and the two tags) of the same single-stranded nucleic acid without synthesizing a new chain or changing a template, the method provides a single-end double-tag sequencing strategy which is different from the conventional double-tag sequencing and can be quickly achieved. Tests have demonstrated that the index hopping frequency in the sequencing result acquired by the method can be down to a level of 1/100,000 or even 1/1,000,000. In a certain specific example, the first sequencing primer, the second sequencing primer, and the third sequencing primer, which respectively include sequences set forth in SEQ ID NOs: 1-3, can well implement the method to give the corresponding sequencing result.

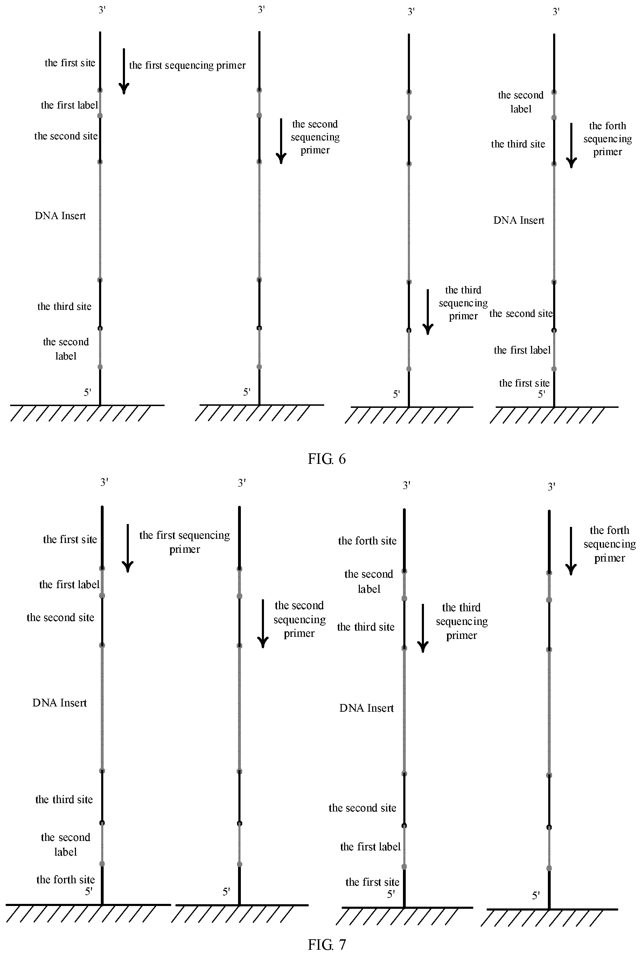

In some certain examples, the single-stranded nucleic acid is a first single-stranded nucleic acid; the surface is further connected with a second single-stranded nucleic acid; the second single-stranded nucleic acid is a complementary strand of the first single-stranded nucleic acid; the second single-stranded nucleic acid is connected via a 5' end and a 5' end of a part thereof complementary to the first sequence with the surface. Specifically, in a certain example, referring to FIG. 6, the method further includes: providing a fourth sequencing primer capable of hybridizing with a 5' end of a part of the second single-stranded nucleic acid complementary to the third site; and hybridizing the fourth sequencing primer with the second single-stranded nucleic acid and placing under a condition suitable for polymerization sequencing to determine at least a part of the sequence of the insert on the single-stranded nucleic acid by extending the fourth sequencing primer, so as to acquire the sequencing result. The order of executing the procedures of extending the first sequencing primer, the second sequencing primer, the third sequencing primer, or the fourth sequencing primer to determine the corresponding sequences is not specified in the embodiment. By designing and jointly using the first sequencing primer, the second sequencing primer, and the third sequencing primer to determine the sequence information of the three parts (one end of the insert and the two tags) of the same single-stranded template (the first single-stranded nucleic acid) and determine the sequence information of the other end of the insert of the complementary single-stranded template, the method provides a double-indexing sequencing strategy which is different from the conventional sequencing. Tests have demonstrated that the index hopping frequency in the sequencing result acquired by the method can be down to a level of 1/100,000 or even 1/1,000,000.

In some other examples, referring to FIG. 7, the single-stranded nucleic acid is a first single-stranded nucleic acid; the surface is further connected with a second single-stranded nucleic acid; the second single-stranded nucleic acid is a complementary strand of the first single-stranded nucleic acid; the second single-stranded nucleic acid is connected with the surface via a 5'end thereof, namely, via a 5' end of a part thereof complementary to the first sequence; the second sequence is a predetermined sequence including the fourth site - the second tag - the third site; the method further includes: providing a third sequencing primer capable of hybridizing with a 5' end of a part of the second single-stranded nucleic acid complementary to the third site; providing a fourth sequencing primer capable of hybridizing with a 5' end of a part of the second single-stranded nucleic acid complementary to the fourth site; hybridizing the third sequencing primer with the second single-stranded nucleic acid and placing under a condition suitable for polymerization sequencing to determine at least a part of the sequence of the insert on the single-stranded nucleic acid by extending the third sequencing primer; and hybridizing the fourth sequencing primer with the second single-stranded nucleic acid and placing under a condition suitable for polymerization sequencing to determine a sequence of the second tag on the single-stranded nucleic acid by extending the fourth sequencing primer, so as to acquire the sequencing result. The order of executing the procedures of extending the first sequencing primer, the second sequencing primer, the third sequencing primer, or the fourth sequencing primer to determine the corresponding sequences is not specified in the embodiment.

By designing and jointly using the first sequencing primer and the second sequencing primer to determine a part of the insert and the first tag from the same end (3' end) of the insert using the first single-stranded nucleic acid as the template, and designing and jointly using the third sequencing primer and the fourth sequencing primer to determine the other part of the insert and the second tag from the same end of the insert of the complementary single-stranded template, the method provides a double-indexing sequencing strategy which is different from the conventional sequencing. Tests have demonstrated that the index hopping frequency in the sequencing result acquired by the method can be down to a level of 1/100,000 or even 1/1,000,000.

In some certain examples, the single-stranded nucleic acid is a first single-stranded nucleic acid; the surface is further connected with a second single-stranded nucleic acid; the second single-stranded nucleic acid is a complementary strand of the first single-stranded nucleic acid; the second single-stranded nucleic acid is connected with the surface via a 5'end thereof, namely, via a 5' end of a part thereof complementary to the first sequence; a library is amplified on the surface to provide the single-stranded nucleic acid; the library includes a plurality of double-stranded nucleic acid molecules formed from a forward strand and a reverse strand that are complementary; the single-stranded nucleic acid includes an identical sequence to the reverse strand.

Library amplification can be achieved on the surface using bridge amplification (bridge PCR; see Patent Publication No. US20050100900A1) or template-walking amplification (see Zhaochun Ma et al., PNAS, 110(35):14320-14323, Aug. 27, 2013).

Specifically, in a certain example, referring to FIG. 2, the amplification includes: melting the library to give an initial template including the forward strand and the reverse strand; providing a plurality of forward amplification primers and reverse amplification primers immobilized to the surface at 5' ends thereof, where the forward amplification primer is capable of hybridizing with a 3' end of the forward strand and the reverse amplification primer is capable of hybridizing with a 3' end of the reverse strand; hybridizing at least a part of the initial template with the forward amplification primer and/or the reverse amplification primer to synthesize a nascent strand complementary to the initial template by extending the forward amplification primer and/or the reverse amplification primer; removing the initial template; and performing bridge amplification by using the nascent strand as a template and the forward amplification primer or the reverse amplification primer as a primer to give a solid substrate having a surface with a plurality of first single-stranded nucleic acids and a plurality of second single-stranded nucleic acids immobilized thereon.

In a specific example, the amplification further includes: after acquiring a solid substrate having a surface with a plurality of first single-stranded nucleic acids and a plurality of second single-stranded nucleic acids immobilized thereon and before the polymerization sequencing, removing the plurality of second single-stranded nucleic acids immobilized on the surface. Thus, individual sequencing template single strands are obtained, which is suitable for situations where the second single-stranded nucleic acid does not need to be determined, such as single-read/single-ended sequencing.

The removal of the second single-stranded nucleic acid can be achieved by providing a cleavage site on the reverse amplification primer and cleaving the strand synthesized using the reverse amplification primer. The cleavage site may be a physical or chemical site of action, such as a photocleavage site, an enzymatic cleavage site, etc.

In one embodiment, the cleavage site is a recognition and action site of an enzyme, such as deoxyuridine (ideoxyU). The uracil base can be removed by using uracil DNA glycosylase (UDG), and can also be cleaved by an enzyme combination (e.g., USER^{™}, New England Biolabs).

Specifically, in some certain examples, the forward amplification primer is an oligonucleotide including poly(N)ₙ - (a complementary part of) the fourth site; the reverse amplification primer is an oligonucleotide including poly(N)ₙ - the cleavage site - (a complementary part of) the first site, or an oligonucleotide including poly(N)ₙ - a complementary part of the first site, where the cleavage site is embedded in (the complementary part of) the first site, N is A, T, C or G, and n is a natural number of not less than 5 and not more than 15. The setting and introduction of poly(N)ₙ in the primer can keep a certain distance between the synthesized template strand and the surface, increase the degree of freedom of the template strand, and facilitate the subsequent biochemical reaction on the surface, including the solid-phase amplification, the enzymatic cleavage, and/or the polymerization sequencing.

In the case that the reverse amplification primer is an oligonucleotide including poly(N)ₙ - the first site and the cleavage site is embedded in the first site, the position of the cleavage site in the reverse amplification primer is not specified in the embodiment. Preferably, the cleavage site is as close as possible to the 5' end of the first site in the primer, such that the part of the reverse amplification primer remaining on the surface after cleavage is as short as possible, thus minimizing the impact on subsequent sequencing.

Optionally, the reverse amplification primer may be provided with a phosphorothioate modification at the 3' end. For example, the -O- in the phosphodiester bond of the first and second nucleotides at the 3' end may be changed to -S-, which is advantageous for stabilizing the primer on the surface and for subsequent sequencing. More specifically, in a certain example, the forward amplification primer has a sequence set forth in SEQ ID NO: 4, and/or the reverse amplification primer has a sequence set forth in SEQ ID NO: 5 or SEQ ID NO: 6 or SEQ ID NO: 7 or SEQ ID NO: 8. Such primers can well achieve the solid-phase amplification, so as to generate a single-stranded template cluster.

Specifically, in other examples, the amplification includes: melting the library to give an initial template including the forward strand and the reverse strand; providing a plurality of forward amplification primers immobilized to the surface at 5' ends thereof, where the forward amplification primer is capable of hybridizing with a 3' end of the forward strand; providing a plurality of free reverse amplification primers, where the reverse amplification primer is capable of hybridizing with a 3' end of the reverse strand; hybridizing at least a part of the forward strand with the forward amplification primer to synthesize a nascent strand complementary to the forward strand by extending the forward amplification primer; removing the forward strand; hybridizing at least a part of the reverse primer with the nascent strand to synthesize a complementary strand of the nascent strand by extending the reverse amplification primer; and performing template-walking amplification by using the nascent strand or the complementary strand of the nascent strand as a template and the reverse amplification primer or the forward amplification primer as a primer to give a solid substrate having a surface with a plurality of first single-stranded nucleic acids immobilized thereon.

In a related example, for the first single-stranded nucleic acid and/or the second single-stranded nucleic acid as sequencing templates, the forward strand and the reverse strand of the library as surface solid-phase amplification templates, the forward and reverse amplification primers of the corresponding solid-phase amplification, and the reverse strand of the library identical to the (first) single-stranded nucleic acid sequence, it will be appreciated that the forward amplification primer binds to the forward strand of the library and extends to synthesize nucleic acid strands including the reverse strand of the library (i.e., the first single-stranded nucleic acid), and the reverse amplification primer binds to the reverse strand of the library and extends to synthesize nucleic acid strands including the forward strand of the library (i.e., the second single-stranded nucleic acid).

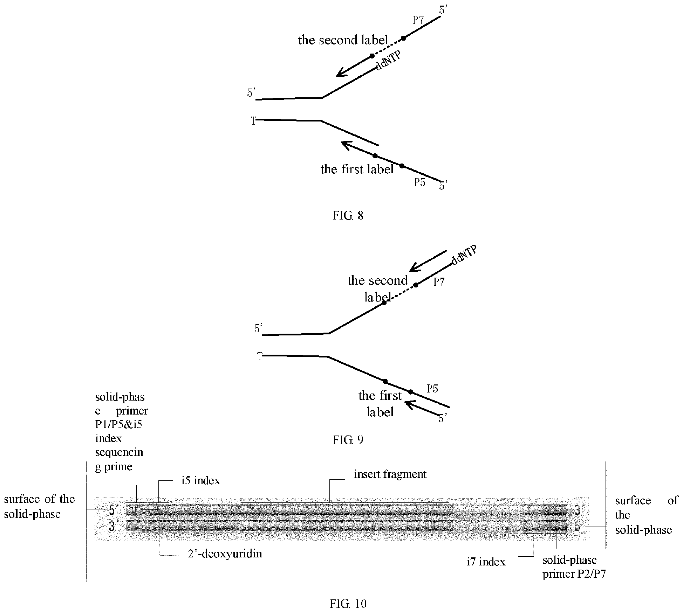

The preparation of the library can be performed according to the library preparation instructions of applicable sequencing platforms. Specifically, in some certain examples, referring to FIG. 1, the preparation of the library is achieved by using an incomplete adapter (including a part of sequence information of the end of the insert of the template under test) in combination with PCR to introduce other sequences to synthesize a complete template under test. Unlike the example shown in FIG. 1, the side of the 5' end (P5 end) of the insert of the prepared library includes a tag (the first tag, or i5 index), and optionally, the side of the 3' end (P7 end) includes a tag (the second tag, or i7 index). Construction of the library may include: providing a double-stranded insert (DNA insert); ligating adapters to the two ends of the insert to give an adapter - insert - adapter double-stranded nucleic acid molecule, where the adapters are double-stranded nucleic acid molecules with predetermined sequences, the adapters consist of a first strand and a second strand that are partially complementary, and a 3' end of the first strand includes a modification (blocker); providing a first amplification primer and a second amplification primer, where a 3' end of the first amplification primer is capable of hybridizing with the 3' end of the first strand of a non-complementary part, a 3' end of the second amplification primer is capable of hybridizing with a 3' end of a complementary strand of the second strand of the non-complementary part, and the second amplification primer includes the first site and the tag; synthesizing a new strand complementary to the adapter - insert - adapter using the first amplification primer; synthesizing a complementary strand of the new strand using the second amplification primer to give a library template including the tag and the first site; and amplifying the library template using the first amplification primer and the second amplification primer to give the library, where a forward strand of the library includes the first strand. An available incomplete adapter (Y adapter) and amplification scheme are shown in FIG. 8, with the 3' end of the first strand blocked with ddNTP and the dotted line representing an optional second tag.

Specifically, in some certain examples, the adapter includes a sequence set forth in SEQ ID NO: 9 and SEQ ID NO: 10, and can be used to construct the library; the sequencing of the library will give a sequencing result with a low index hoping level.

Accordingly, the first amplification primer and the second amplification primer may include sequences set forth in SEQ ID NO: 11 and SEQ ID NO: 12 or sequences set forth in SEQ ID NO: 11 and SEQ ID NO: 13, respectively. Thus, the method is beneficial to the efficient preparation of the library.

In some other examples, the preparation of the library is achieved by using an intact adapter (including all sequence information of the end of the insert of the template under test), including: providing a double-stranded insert; ligating adapters to the two ends of the insert to give an adapter - insert - adapter double-stranded nucleic acid molecule, where the adapters are double-stranded nucleic acid molecules with predetermined sequences, the adapters consist of a first strand and a second strand that are partially complementary, the second strand of a non-complementary part includes the tag and the first site, and a 3' end of the first strand includes a modification; optionally, providing a first amplification primer and a second amplification primer, where the first amplification primer is capable of hybridizing with the 3' end of the first strand of a non-complementary part, and the second amplification primer is capable of hybridizing with a 3' end of a complementary strand of the second strand of the non-complementary part; and optionally, amplifying the adapter - insert - adapter using the first amplification primer and the second amplification primer to give the library, where a forward strand of the library includes the first strand. An available intact adapter (Y adapter) and amplification scheme are shown in FIG. 9, with the 3' end of the first strand blocked with ddNTP and the dotted line representing an optional second tag.

It will be appreciated that the nucleic acid molecules to which the intact adapters are ligated in this example are referred to as a library, and the subsequent solid-phase amplification and sequencing of the library can be conducted without further amplification, i.e., providing the first amplification primer and the second amplification primer and amplifying the ligation products using the amplification primers in this example, are optional steps.

When using the adapter with a modification at the end, particularly a designated strand with a modification at the 3' end, to construct a library, the 3' end of the designated strand cannot bind to nucleotides and cannot be extended, which is beneficial to the further reduction of index hoping. In some certain examples, the modification may be selected from at least one of an amino modification, a dideoxynucleotide modification, and a PEG modification, so as to block the 3' end of the designated strand.

According to an embodiment of the present disclosure, further provided is a kit for implementing the sequencing method according to any one of the above embodiments, including the solid substrate and the first sequencing primer. It will be appreciated that in some certain examples, a second sequencing primer, a third sequencing primer, and/or sequences for library construction (adapters, amplification primers, etc.), and the like, are also included.

According to an embodiment of the present disclosure, further provided is a system capable of implementing the sequencing method according to any one of the above examples, which is an automatic device for implementing any one of the above sequencing methods, including: a mechanical mechanism for holding the solid substrate; a liquid path structure connected with the mechanical mechanism for introducing a first sequencing primer, DNA polymerase and the like into the solid substrate, including a pump; a control unit connected with the mechanical mechanism and the liquid path structure for enabling the hybridization and/or enabling the presence of substances on the solid substrate in an environment suitable for polymerization sequencing; and the like.

According to an embodiment of the present disclosure, further provided is a computer-readable storage medium configured for storing a program executed by a computer, and executing the program includes implementing the sequencing method according to any of the above embodiments. The computer-readable storage medium may include: read-only memory, random access memory, magnetic disk, optical disk, or the like.

An embodiment of the present disclosure further provides a computer product, including a memory for storing data and a control system, where the data stored in the memory further includes a computer-executable program, and the control system executing the computer-executable program includes implementing the sequencing method according to any one of the above embodiments.

The technical solutions of the present disclosure are described in detail by the following examples, and it will be appreciated that the examples are only exemplary and should not be construed as limiting the scope of the present disclosure. The materials, reagents, sequences, and the like mentioned in the examples were prepared or synthesized in-house, or commercially available, unless otherwise specified.

A plurality of nucleic acid samples for multiplex sequencing were set: *Escherichia coli*_ATCC8733, human_gDNA, and Phix174_gDNA library. To test the index hopping level for mixed sequencing of complex or extreme multiple samples using the exemplified solutions, nucleic acids from the same sample were divided into multiple aliquots to construct multiple different libraries, and the index hopping was evaluated by cross-alignment. It will be appreciated that the mixed sequencing of multiple samples from the same species cannot distinguish the samples according to the alignment results when index hopping occurs in the multiplex sequencing of the samples, i.e., when the mixed data cannot be accurately distributed to the corresponding samples. As such, compared with the real situation (where different samples generally have differences at the level of nucleic acid sequence), this is an extreme case, and can reflect the influence of the exemplified solutions on the level of index hopping.

Here, the E. *coli_*ATCC8733 library a (with tag a), E. *coli_*ATCC8733 library b (with tag b), and E. *coli_*ATCC8733 library c (with tag c) were constructed by ligating three different tags (a, b, and c) to the E. *coli_*ATCC8733 sample, representing three different samples.

### Example 1

In combination with a commercially available multiplex library construction kit (e.g., VAHTS^{™} Multiplex Oligos Set 2 for Illumina^{®}, Vazyme) and self-designed sequences (adapters, etc.), the samples were subjected to the following library construction with reference to the kit instructions to give the E. *coli_ATCC8733* library a, *E. coli*_ATCC8733-2 library b, E. *coli_*ATCC8733 library c, human_gDNA library, and Phix174_gDNA reference library.

The construction of the libraries of the samples includes:
1) End repair and addition of dA: A DNA polymerase such as Klenow was added for the end repair of the fragmented genomic DNA fragments (inserts). The 5' overhangs were filled in, while the 3' overhangs were cleaved. A Klenow fragment enzyme was used to add A at the 3' end and T4 PNK was used at the 5' end for phosphorylation.
2) Addition of adapters at the ends: Adapters, which may be adapter 1 or adapter 2 consisting of the following sequences, were ligated to the two ends of the insert based on TA sticky end ligation using DNA ligase. Adapter 1 and adapter 2 are identical in sequence, but different in that the sequence set forth in SEQ ID NO: 6 of adapter 1 is in a native state at the 3' end, while the corresponding strand of adapter 2 carries a designated modification, which prohibits the addition of nucleotides.

First set of sequence (adapter 1):
   S1: 5'-ACACTCTTTCCCTACACGACGCTCTTCCGATCT-3' (SEQ ID NO: 10)
   S2: 5'-GATCGGAAGAGCACACGTCTGAACTCCAGTC-3' (SEQ ID NO: 9)
Second set of sequence (adapter 2):
   S1: 5'-ACACTCTTTCCCTACACGACGCTCTTCCGATCT-3' (SEQ ID NO: 10)
   S2-C6: 5'-GATCGGAAGAGCACACGTCTGAACTCCAGTC-3' modification (SEQ ID NO: 9)

The modification at the 3' end of S2-C6 strand in the second set may be one or more of an amino modification, a dideoxynucleotide modification, and a PEG modification, and is intended to block the end and prevent the polymerization or extension reaction at the end. 3) Amplification: The ligation product was amplified by using PCR primers with indexes to give a library with the indexes of a certain concentration.
i5 universal primer (second amplification primer, including P5 sequence):
   5'-AATGATACGGCGACCACCGAGATCTACACTCTTTCCCTACACGACGCTCTTCCGATCT-3' (SEQ ID NO: 12), or
PCR primer with i5 index introduced (second amplification primer, including P5 sequence): 
5'-AATGATACGGCGACCACCGAGATCTACAC[i5Index]ACACTCTTTCCCTACACGACGCTCTTCCGAT
CT-3' (SEQ ID NO: 13);
PCR primer with i7 index introduced (first amplification primer, including P7 sequence): 5'-CAAGCAGAAGACGGCATACGAGAT[i7Index]GTGACTGGAGTTCAGACGTGTGCTCTTCCGATC-3' (SEQ ID NO: 11), where [i5Index] and [i7 Index] are tag/index sequences.

According to the above-mentioned adapters, and the number and positions of introduced tags, the library construction method of this example may give an adapter 1 i7 single-tag library, an adapter 1 i5/i7 double-tag library, an adapter 2 i7 single-tag library, and an adapter 2 i5/i7 double-tag library for each sample.

In addition, using SEQ ID NO: 11 without i7 index and SEQ ID NO: 13, adapter 1 or adapter 2 i5 single-tag libraries of the samples can be constructed.

The amplified libraries were mixed. The mixture library (multiplex library) was loaded for high-throughput sequencing by using an MiSeq, HiSeq or NextSeq sequencing platform of Illumina, an MGISEQ or DNBSEQ sequencing platform of BGI, or a GenoLab^{™} sequencing platform of Genemind Biosciences, Co., Ltd.

### Example 11

The adapter 1 double-tag libraries and the adapter 2 double-tag libraries of the samples were constructed according to the above procedures. The adapter 1 double-tag libraries of the samples and the adapter 2 double-tag libraries of the samples were separately mixed to give the adapter 1 mixture library and the adapter 2 mixture library. The configuration and proportions of the double-tag libraries are shown in Table 1.

**Table 1**

| Species/sample | i5 index | i7 index | Proportion (%) |
|---|---|---|---|
| *E. coli_*ATCC8733 library a | CTCTCTAT | TAGGCATG | 26.67 |
| *E. coli_*ATCC8733 library b | TATCCTCT | GGACTCCT | 26.67 |
| *E. coli_*ATCC8733 library c | GTAAGGAG | TCCTGAGC | 26.67 |
| Human gDNA library | ACTGCATA | AGGCAGAA | 10 |
| Phix174_gDNA library | AAGGAGTA | CTCTCTAC | 10 |

### Example 111

The mixture libraries (sometimes abbreviated as the libraries) were loaded onto the chips according to the sequencing instructions of the sequencing platform. For example, according to the following procedures, the library was denatured and hybridized to a chip, single-stranded libraries were amplified into clusters on the chip surface, and the polymerization sequencing was performed.
1) Sequencing sample preparation (denaturation/melting of the mixture library, hybridization of single-stranded libraries introduced into the chip with solid-phase probes)
The library stock solution was diluted to 4 nM^{#} with pre-cooled library diluent (10 mM Tris-HCl (pH 8.5) + 0.1% Tween 20) (libraries with a concentration of 4 nM were not diluted), and then subjected to library denaturation as in Table 2 to formulate a 20 pM library:

**Table 2**

| Reagents | Volume (µL) |
|---|---|
| 0.2 M NaOH | 5 |
| 4 nM Library (mixture library) | 5 |
| After mixing, the library was let stand at room temperature for 5 min for denaturation. After completion of denaturation, the reaction was stopped by placing on ice and addition of 200 mM Tris-HCl (pH 7.5). | |
| 200 mMTris-HCl (pH 7.5) | 5 |
| Hybridization solution | 985 |
| Total library 20 pM | 1000 |

| | |
|---|---|
| ^{#} If the sample concentration is lower than 4 nM but higher than 0.3 nM, denaturation can still be performed. However, it should be noted that the final concentration of NaOH shall be kept at 0.1 M during the 5-min denaturation. | |

2) Referring to FIG. 2, the amplification into clusters was performed on a sequencing platform, specifically including: a) a single-stranded library was hybridized with a substrate having two primers/probes immobilized on the surface thereof, where the library included two complementary single strands, a forward strand and a reverse strand, corresponding to the amplification primers constructed from the library of example 1, which are referred to as P7 solid-phase primer and P5 solid-phase primer herein and can hybridize with the 3' ends of the forward strand and the reverse strand, respectively; b) the P7 or P5 solid-phase primer was extended to synthesize the complementary strands of the single-stranded library; c) the double strands were denatured to give new single-stranded templates (complementary strands); d) the mixture was annealed to allow the complementary strands to hybridize with the P5 or P7 solid-phase primer; e) the P7 or P5 solid-phase primer was extended to synthesize new complementary strands. Procedures c) and d) were repeated for 35 cycles of denaturation-annealing-extension to give the clusters. It will be appreciated that a cluster substantially corresponds to or represents a library molecule/insert, and that the clustering process amplifies the signal from a single target sequence/insert, facilitating subsequent acquisition of polymerization reaction signals from the clusters/single libraries.
3) Further preparation for sequencing: A combination of enzyme reagents was added to act on the cleavage site on the P5 probe, so as to remove the (library) forward strand template, such that only one template was left on the surface as the sequencing template (library reverse strand).

### Example 1111

The i5 single-index sequencing method or i5 index+i7 index sequencing method was achieved on the basis of single-read sequencing only by using special solid-phase amplification primers.

"Solid-phase amplification" refers to any polynucleotide amplification reaction conducted on or in association with a solid support such that all or part of the amplification products are immobilized on the solid support as they are formed. In particular, the term includes solid-phase polymerase chain reaction (solid-phase PCR) and solid-phase isothermal amplification, and refers to a reaction similar to the standard solution-phase amplification except that one or both of the forward amplification primer and the reverse amplification primer are immobilized on a solid support. Primers used for solid-phase amplification were preferably immobilized by single-site covalent attachment to the solid support at or near the 5' end of the primer, leaving the template-specific portion of the primer free to anneal to its cognate template and the 3' hydroxyl group free for primer extension.

In this example, referring to the library structure illustrated in FIG. 10 and the manner of attachment of the library template to the surface after solid-phase amplification, to achieve the single-end i5 index sequencing, the sequences of the primers covalently attached to the solid support for solid-phase amplification were:
Solid-phase primer 1: 5'-TTTTTTTTT/ideoxyU/AATGATACGGCGACCACCGAGATCTACAC-3' (SEQ ID NO: 5, solid-phase primer PS), or
TTTTTTTTTTAA/ideoxyU/GATACGGCGACCACCGAGATCTACA*C (SEQ ID NO: 6, solid-phase primer PS), or
TTTTTTTTTTAATGA/ideoxyU/ACGGCGACCACCGAGATCTACA*C (SEQ ID NO: 7, solid-phase primer PS), or
TTTTTTTTTTAATGATACGGCGACCACCGAGA/ideoxyU/CTACA*C (SEQ ID NO: 8, solid-phase primer P5);

Solid-phase primer 2: 5'-TTTTTTTTTTCAAGCAGAAGACGGCATACGAGAT-3' (SEQ ID NO: 4, solid-phase primer P7), where the /idexoxyU/ stands for 2'-deoxyuridine (dUTP). As a primer for solid-phase amplification, the double strands of the solid-phase amplification product carried the modification/site near the 5' end, and were capable of being cleaved by USER^{™} (NEB Cat# M5505D) to remove all the amplified strands of the solid-phase primer 1 including the modification, such that the complementary single-stranded DNA of the amplified strand of the solid-phase primer 1 remained on the surface, facilitating the hybridization of the i5 index primer and the complementary strand of the solid phase primer 1 and the sequencing. A site indicated by * represents that the -O- in the phosphodiester bond at that site was optionally substituted by sulfur.

The sequence of the i5 index sequencing primer (a sequencing primer capable of hybridizing with the 3' end of the reverse strand of the library designed according to the P5 solid-phase primer or the P5 end sequence, i.e., the sequencing primer for reading the i5 index) is:
5'-GATACGGCGACCACCGAGATCTACAC-3' (SEQ ID NO: 1);

Comparing the sequences of the solid-phase primer 1 and the i5 index sequencing primers, it can be seen that the two include the same sequence. Referring to FIG. 10, according to the library structure, the i5 index sequencing primer was hybridized before sequencing, and the read signal reflected the information of the i5 index sequence.

Therefore, using the solid-phase primer 1 and the solid-phase primer 2 as above, the amplification cluster generated by solid-phase amplification can be subjected to i5 index sequencing by the i5 index sequencing primer given in this example after sequencing the fragment under test using the sequencing primer 1. Optionally, i7 index sequencing can be performed by hybridization and extension of the i7 index sequencing primer in addition to i5 index primer sequencing. There is no requirement to order of performing the i5 index sequencing and the i7 index sequencing, as shown in FIG. 5.

The sequence of the i7 index sequencing primer (the sequencing primer for reading the i7 index) is: 5'-GATCGGAAGAGCACACGTCTGAACTCCAGTCAC-3' (SEQ ID NO: 2)

The sequence of the sequencing primer 1 (the sequencing primer for reading the insert) is: 5'-ACACTCTTTCCCTACACGACGCTCTTCCGATCT-3' (SEQ ID NO: 3).

Corresponding sequencing primers were introduced into the chip, and the SBS sequencing was performed on one end of the insert, the i5 tag, and the i7 tag. For example, the insert was sequenced using two-color sequencing (two channel), including: a) a nucleotide sequence set forth in SEQ ID NO: 3 was introduced to hybridize the sequencing primer with a sequencing template; b) four reversible terminators (four modified nucleotides with detectable labels such as fluorescent molecules that can inhibit the binding of other nucleotides to the next position of the template under test), and under the action of polymerase, the modified nucleotides were allowed to bind to the sequencing primer/template under tested; c) the fluorescent molecules were excited to emit light, and the light emitting signals were acquired, for example, by photographing, to give images; d) a cleavage reagent was introduced to remove the fluorescent molecules and inhibitory groups on the modified nucleotide bound to the sequencing primer/template under test. Procedures b) to d), which were defined as a cycle of sequencing, were performed multiple times, and the bases were called based on image information to determine at least a part of the sequence of the insert.

The nascent strand (the strand including the sequencing primer) was then melted and removed, and the i5 tag was sequenced by adding the corresponding sequencing primer, e.g., the i5 index sequencing primer. Based on the length of the i5 tag, an appropriate number of sequencing cycles can be set to achieve the determination of the sequence of the i5 tag.

### Example 10

According to the unique correspondence between the tag and the sample, the sequencing data of the mixture library from the sequencing platform were demultiplexed/distributed to give the sequencing result of each sample in the mixture library. The sequencing data after the demultiplexing can be processed according to a known method, for example, by using the Bowtie software (Langmead B. Aligning Short Sequencing Reads with Bowtie. Current Protocols in Bioinformatics Vol 32, Iss 1, 2010, pp 11.7.1-11.7.14.) widely used in the art for comparison, and the data processing and analysis workflow can be adjusted according to differences in operating system and the like by referring to Bowtie help files.

The sequencing data of the adapter 1 i7 index libraries and the adapter 2 i7 index libraries of the mixture sample were demultiplexed by using Bowtie, and the data obtained by demultiplexing were cross-aligned to reference sequences of the three species. The alignment results are shown in Table 3.

**Table 3**

| *E. coli* reference sequence | | | | |
|---|---|---|---|---|
| | Sample/library | Mapped rate% | Total reads | Mapped reads |
| Adapter set 2 mixture library | *E*. *coli_*ATCC8733 library a | 89.51 | 58939488 | 52754402 |
| | *E*. *coli_*ATCC8733 library b | 89.65 | 51145546 | 45854478 |
| | *E*. *coli_*ATCC8733 library c | 90.30 | 46955186 | 42402294 |
| | Human gDNA library | 0.629 | 26961519 | 169717 |
| | Phix174_gDNA library | 0.820 | 22279730 | 182618 |
| Adapter set 1 mixture library | *E*. *coli_*ATCC8733 library a | 91.77 | 57083283 | 52383599 |
| | *E*. *coli_*ATCC8733 library b | 91.96 | 52420823 | 48204349 |
| | *E*. *coli_*ATCC8733 library c | 92.24 | 68081640 | 62801480 |
| | Human gDNA library | 0.840 | 26209558 | 220055 |
| | Phix174_gDNA library | 1.175 | 23166419 | 272212 |

| Human reference sequence (HG19) | | | | |
|---|---|---|---|---|
| | Sample/library | Mapped rate% | Total reads | Mapped reads |
| Adapter set 2 mixture library | *E*. *coli_*ATCC8733 library a | 0.120 | 58939488 | 70556 |
| | *E*. *coli_*ATCC8733 library b | 0.128 | 51145546 | 65461 |
| | *E*. *coli_*ATCC8733 library c | 0.085 | 46955186 | 40043 |
| | Human gDNA library | 85.55 | 26961519 | 23066860 |
| | Phix174_gDNA library | 0.120 | 22279730 | 26640 |
| Adapter set 1 mixture library | *E*. *coli_*ATCC8733 library a | 0.126 | 57083283 | 71936 |
| | *E*. *coli_*ATCC8733 library b | 0.136 | 52420823 | 71062 |
| | *E*. *coli_*ATCC8733 library c | 0.096 | 68081640 | 65181 |
| | Human gDNA library | 87.48 | 26209558 | 22927408 |
| | Phix174_gDNA library | 0.133 | 23166419 | 30756 |

| PhiX174 reference sequence | | | | |
|---|---|---|---|---|
| | Sample/library | Mapped rate% | Total reads | Mapped reads |
| Adapter set 2 mixture library | *E*. *coli_*ATCC8733 library a | 0.104 | 58939488 | 61156 |
| | *E*. *coli_*ATCC8733 library b | 0.113 | 51145546 | 57927 |
| | *E*. *coli_*ATCC8733 library c | 0.074 | 46955186 | 34817 |
| | Human gDNA library | 0.094 | 26961519 | 25225 |
| | Phix174_gDNA library | 85.48 | 22279730 | 19045047 |
| Adapter set 1 mixture library | *E*. *coli_*ATCC8733 library a | 0.129 | 57083283 | 73369 |
| | *E*. *coli_*ATCC8733 library b | 0.141 | 52420823 | 74086 |
| | *E*. *coli_*ATCC8733 library c | 0.100 | 68081640 | 68109 |
| | Human gDNA library | 0.112 | 26209558 | 29211 |
| | Phix174_gDNA library | 87.40 | 23166419 | 20247772 |

As can be seen from Table 2, the index hopping level for library construction using adapter 2 with the modification at the end was about 20% lower than that with adapter 1. Therefore, using modified adapters for library construction can reduce the index hopping level to a certain extent.

### Example 100

According to the library construction process and the library structure, the free adapter at the end of P5 cannot be hybridized with the excessive P5 primers (solid-phase primer) and extended, and it is supposed that the possibility of index hopping may be reduced when the i5 index is used alone for demultiplexing.

The following primers were synthesized as the sequencing primers:
i5 index sequencing primer 5'-GATACGGCGACCACCGAGATCTACAC-3' (SEQ ID NO: 1)
i7 index sequencing primer 5'-GATCGGAAGAGCACACGTCTGAACTCCAGTCAC-3' (SEQ ID NO: 2)

Double-unit sequencing (two physically isolated regions/surfaces on the same reactor, e.g., two channels on a chip) was performed on an SBS sequencing platform such as GenoLab^{™} platform on an adapter 1 or adapter 2 double-tag library. On one unit, the i5 index sequencing primer was used for index sequencing and the sequencing data were demultiplexed according to the i5 index; on the other unit, the i7 index sequencing primer was used for sequencing and the sequencing data was demultiplexed according to i7. The resultant sequencing data were cross-aligned with reference sequences of the three species. The alignment results are shown in Tables 4 and 5:

**Table 4**

| Sequencing data demultiplexing according to i5 index | | | |
|---|---|---|---|
| *E. coli* reference sequence | | | |
| Sample | Mapped rate% | Total reads | Mapped reads |
| *E*. *coli_*ATCC8733 library a | 94.25 | 8865542 | 8355733 |
| *E*. *coli_*ATCC8733 library b | 94.11 | 7475166 | 7034805 |
| *E*. *coli_*ATCC8733 library c | 92.99 | 7495031 | 6969369 |
| Human gDNA library | 0.084 | 4554665 | 3804 |
| Phix174_gDNA library | 0.040 | 3490824 | 1393 |

| Phix174_gDNA reference sequence | | | |
|---|---|---|---|
| Sample | Mapped rate% | Total reads | Mapped reads |
| *E*. *coli_*ATCC8733 library a | 0.017 | 8865542 | 1528 |
| *E*. *coli_*ATCC8733 library b | 0.027 | 7475166 | 2019 |
| *E*. *coli_*ATCC8733 library c | 0.005 | 7495031 | 350 |
| Human gDNA library | 0.014 | 4554665 | 651 |
| Phix174_gDNA library | 94.86 | 3490824 | 3311330 |

| Human reference sequence | | | |
|---|---|---|---|
| Sample | Mapped rate% | Total reads | Mapped reads |
| *E*. *coli*_ATCC8733 library a | 0.006 | 8865542 | 513 |
| *E*. *coli_*ATCC8733 library b | 0.034 | 7475166 | 254 |
| *E*. *coli_*ATCC8733 library c | 0.022 | 7495031 | 1662 |
| Human gDNA library | 96.29 | 4554665 | 4385740 |
| Phix174_gDNA library | 0.033 | 3490824 | 1158 |

**Table 5**

| Sequencing data demultiplexing according to i7 index | | | |
|---|---|---|---|
| *E. coli* reference sequence | | | |
| Sample/library | Mapped rate% | Total reads | Mapped reads |
| E. *coli_*ATCC8733 library a | 91.67 | 2444594 | 2240935 |
| E. *coli_*ATCC8733 library b | 91.99 | 2317773 | 2132035 |
| E. *coli_*ATCC8733 library c | 91.73 | 2995699 | 2748041 |
| Human gDNA library | 0.974 | 1063018 | 10353 |
| Phix174_gDNA library | 1.317 | 926775 | 12205 |

| Human reference sequence (HG19) | | | |
|---|---|---|---|
| Sample/library | Mapped rate% | Total reads | Mapped reads |
| *E*. *coli_*ATCC8733 library a | 0.138 | 2444594 | 3388 |
| *E*. *coli_*ATCC8733 library b | 0.105 | 2317773 | 3259 |
| *E*. *coli_*ATCC8733 library c | 0.105 | 2995699 | 3136 |
| Human gDNA library | 91.04 | 1063018 | 967773 |
| Phix174_gDNA library | 0.149 | 926775 | 1385 |

| PhiX174 reference sequence | | | |
|---|---|---|---|
| Sample/library | Mapped rate% | Total reads | Mapped reads |
| *E*. *coli_*ATCC8733 library a | 0.118 | 2444594 | 2876 |
| *E*. *coli_*ATCC8733 library b | 0.125 | 2317773 | 2901 |
| *E*. *coli_*ATCC8733 library c | 0.085 | 2995699 | 2531 |
| Human gDNA library | 0.117 | 1063018 | 1246 |
| Phix174_gDNA library | 90.39 | 926775 | 837735 |

As can be seen from the results in Tables 4 and 5, the index hopping frequency using i5 index demultiplexing is one to two orders of magnitude lower than that of i7 index demultiplexing.

### Example 1000

The sequencing data were demultiplexed using the i5 index and the i7 index to determine the frequency of index hopping.

The following primers were synthesized as the sequencing primers:
i5 index sequencing primer 5'-GATACGGCGACCACCGAGATCTACAC-3' (SEQ ID NO: 1)
i7 index sequencing primer 5'-GATCGGAAGAGCACACGTCTGAACTCCAGTCAC-3' (SEQ ID NO: 2).

Single-ended double-index sequencing was performed on the mixture library on an SBS sequencing platform such as GenoLab^{™} platform. The sequencing data were demultiplexed using the i5 index and the i7 index, and the demultiplexed sequencing data were cross-aligned with reference sequences of the three species. The alignment results are shown in Table 6 below:

**Table 6**

| Sequencing data demultiplexing according to i5 index and i7 index | | | |
|---|---|---|---|
| *E. coli* reference sequence | | | |
| Sample/library | Mapped rate% | Total reads | Mapped reads |
| *E*. *coli_*ATCC8733 library a | 93.78 | 3064972 | 2874361 |
| *E*. *coli_*ATCC8733 library b | 94.11 | 2741670 | 2580295 |
| *E*. *coli_*ATCC8733 library c | 94.05 | 3396415 | 3194362 |
| Human gDNA library | 0.052 | 1437585 | 748 |
| Phix174_gDNA library | 0.026 | 1114393 | 290 |

| Human genome reference sequence (HG19) | | | |
|---|---|---|---|
| Sample/library | Mapped rate% | Total reads | Mapped reads |
| E. *coli_*ATCC8733 library a | 0.004 | 3064972 | 123 |
| E. *coli_*ATCC8733 library b | 0.005 | 2741670 | 137 |
| E. *coli_*ATCC8733 library c | 0.002 | 3396415 | 68 |
| Human gDNA library | 95.88 | 1437585 | 1378400 |
| Phix174_gDNA library | 0.004 | 1114393 | 45 |

| PhiX174 reference sequence | | | |
|---|---|---|---|
| Sample/library | Mapped rate% | Total reads | Mapped reads |
| *E*. *coli_*ATCC8733 library a | 0.001 | 3064972 | 31 |
| *E*. *coli_*ATCC8733 library b | 0.001 | 2741670 | 27 |
| *E*. *coli_*ATCC8733 library c | 0.001 | 3396415 | 34 |
| Human gDNA library | 0.005 | 1437585 | 72 |
| Phix174_gDNA library | 94.17 | 1114393 | 1049502 |

As can be seen from Table 6, the index hopping frequency in sequencing data demultiplexing using the i5 and i7 indexes is down to 1/1,000,000. Comparing Table 6 with Table 5 in example 100, it can be seen that the index hopping frequency in the sequencing data demultiplexing using i5 and i7 indexes is one to two orders of magnitude lower than that of i7 index demultiplexing.

In the description of this specification, the description of the terms "one embodiment", "some embodiments", "schematic embodiments", "examples", "certain examples", "specific examples", or the like, means that the particular features, structures, materials, or characteristics described with reference to the embodiment or example are included in at least one embodiment or example of the present disclosure. In this specification, the schematic description of the aforementioned terms does not necessarily refer to the same embodiment or example. Moreover, the particular features, structures, materials, or characteristics described may be combined in any embodiment or example in any appropriate manner.

In addition, each functional unit in each embodiment in the specification may be integrated into one processing module, or each unit may be physically present alone, or two or more units may be integrated into one module. The integrated module described may be implemented in the form of hardware or in the form of a software functional module. The integrated module may also be stored in a computer-readable storage medium if it is implemented in the form of a software functional module and is sold or used as standalone products.

Although the embodiments of the present disclosure have been illustrated and described above, it will be appreciated that the aforementioned embodiments are exemplary and should not be construed as limiting the present disclosure, and that those of ordinary skills in the art can make changes, modifications, replacements, and variations to such embodiments, without departing from the scope of the present disclosure.

## Claims

1. A sequencing method, comprising:
providing a solid substrate having a surface connected with a plurality of single-stranded nucleic acids, wherein 5' ends of the single-stranded nucleic acids are connected to the surface, the single-stranded nucleic acids are polynucleotides comprising an insert - a first sequence, the insert is a nucleic acid sequence from a sample under test, the first sequence is a predetermined sequence comprising a tag - a first site, and the tag is a predetermined sequence with specificity to the sample under test;
providing a first sequencing primer capable of hybridizing with a 5' end of the first site; and
hybridizing the first sequencing primer with the single-stranded nucleic acid and placing under a condition suitable for polymerization sequencing to determine a part of the sequence of the single-stranded nucleic acid by extending the first sequencing primer, so as to acquire a sequencing result.

2. The method according to claim 1, wherein the sequencing result comprises a read comprising sequence information of the tag and at least a part of the insert.

3. The method according to claim 2, wherein the length of the read is no less than four times the length of the tag.

4. The method according to claim 1, wherein the first sequence is a predetermined sequence comprising a second site - the tag - the first site, and the method further comprises:
providing a second sequencing primer capable of hybridizing with a 5' end of the second site; and
hybridizing the second sequencing primer with the single-stranded nucleic acid and placing under a condition suitable for polymerization sequencing to determine at least a part of the sequence of the insert on the single-stranded nucleic acid by extending the second sequencing primer, so as to acquire the sequencing result.

5. The method according to claim 4, wherein the sequencing result comprises a first read and a second read; the first read comprises sequence information of the tag, and the second read comprises sequence information of at least a part of the insert.

6. The method according to claim 5, wherein the single-stranded nucleic acid is a polynucleotide comprising a second sequence - the insert - the first sequence, and the second sequence is a predetermined sequence comprising a third site.

7. The method according to claim 6, wherein the single-stranded nucleic acid is covalently attached to the surface of the solid substrate via a 5' end of the second sequence.

8. The method according to claim 6 or 7, wherein the tag is a first tag, the second sequence is a predetermined sequence comprising a second tag - the third site or a predetermined sequence comprising a fourth site - the second tag - the third site, and the second tag is a predetermined sequence with specificity to the sample under test.

9. The method according to claim 8, further comprising:
providing a third sequencing primer capable of hybridizing with a 5' end of the third site; and
hybridizing the third sequencing primer with the single-stranded nucleic acid and placing under a condition suitable for polymerization sequencing to determine the sequence of the second tag on the single-stranded nucleic acid by extending the third sequencing primer, so as to acquire the sequencing result.

10. The method according to claim 9, wherein the sequencing result further comprises a third read comprising sequence information of the second tag.

11. The method according to any one of claims 8 to 10, wherein the single-stranded nucleic acid is a first single-stranded nucleic acid; the surface is further connected with a second single-stranded nucleic acid; the second single-stranded nucleic acid is a complementary strand of the first single-stranded nucleic acid; the second single-stranded nucleic acid is connected via a 5' end of a part thereof complementary to the first sequence with the surface.

12. The method according to claim 11, further comprising:
providing a fourth sequencing primer capable of hybridizing with a 5' end of a part of the second single-stranded nucleic acid complementary to the third site; and
hybridizing the fourth sequencing primer with the second single-stranded nucleic acid and placing under a condition suitable for polymerization sequencing to determine at least a part of the sequence of the insert on the single-stranded nucleic acid by extending the fourth sequencing primer, so as to acquire the sequencing result.

13. The method according to claim 8, wherein the single-stranded nucleic acid is a first single-stranded nucleic acid; the surface is further connected with a second single-stranded nucleic acid; the second single-stranded nucleic acid is a complementary strand of the first single-stranded nucleic acid; the second single-stranded nucleic acid is connected via a 5' end of a part thereof complementary to the first sequence with the surface; the second sequence is a predetermined sequence comprising the fourth site - the second tag - the third site; the method further comprises:
providing a third sequencing primer capable of hybridizing with a 5' end of a part of the second single-stranded nucleic acid complementary to the third site;
providing a fourth sequencing primer capable of hybridizing with a 5' end of a part of the second single-stranded nucleic acid complementary to the fourth site;
hybridizing the third sequencing primer with the second single-stranded nucleic acid and placing under a condition suitable for polymerization sequencing to determine at least a part of the sequence of the insert on the single-stranded nucleic acid by extending the third sequencing primer; and
hybridizing the fourth sequencing primer with the second single-stranded nucleic acid and placing under a condition suitable for polymerization sequencing to determine the sequence of the second tag on the single-stranded nucleic acid by extending the fourth sequencing primer, so as to acquire the sequencing result.

14. The method according to any one of claims 6 to 10, wherein the single-stranded nucleic acid is a first single-stranded nucleic acid; the surface is further connected with a second single-stranded nucleic acid; the second single-stranded nucleic acid is a complementary strand of the first single-stranded nucleic acid; the second single-stranded nucleic acid is connected via a 5' end of a part thereof complementary to the first sequence with the surface;
a library is amplified on the surface to provide the single-stranded nucleic acid; the library comprises a plurality of double-stranded nucleic acid molecules formed from a forward strand and a reverse strand that are complementary; the single-stranded nucleic acid comprises an identical sequence to the reverse strand.

15. The method according to claim 14, wherein the amplification is bridge amplification or template-walking amplification.

16. The method according to claim 15, wherein the amplification comprises:
melting the library to give an initial template comprising the forward strand and the reverse strand;
providing a plurality of forward amplification primers and reverse amplification primers immobilized to the surface at 5' ends thereof, wherein the forward amplification primer is capable of hybridizing with a 3' end of the forward strand and the reverse amplification primer is capable of hybridizing with a 3' end of the reverse strand;
hybridizing at least a part of the initial template with the forward amplification primer and/or the reverse amplification primer to synthesize a nascent strand complementary to the initial template by extending the forward amplification primer and/or the reverse amplification primer;
removing the initial template; and
performing bridge amplification by using the nascent strand as a template and the forward amplification primer or the reverse amplification primer as a primer to give a solid substrate having a surface with a plurality of first single-stranded nucleic acids and a plurality of second single-stranded nucleic acids immobilized thereon.

17. The method according to claim 16, further comprising: removing the plurality of second single-stranded nucleic acids immobilized on the surface before the polymerization sequencing.

18. The method according to claim 16 or 17, wherein the reverse amplification primer comprises a cleavage site, and the cleavage site is an enzyme action site.

19. The method according to claim 18, wherein the cleavage site is deoxyuridine.

20. The method according to claim 18 or 19, wherein the reverse amplification primer is an oligonucleotide comprising poly(N)ₙ - the cleavage site - a complementary part of the first site, or,
the reverse amplification primer is an oligonucleotide comprising poly(N)ₙ - a complementary part of the first site, the cleavage site is embedded in the complementary part of the first site, N is A, T, C or G, and n is a natural number of not less than 5 and not more than 15.

21. The method according to claim 20, wherein the forward amplification primer has a sequence set forth in SEQ ID NO: 4, and/or the reverse amplification primer has a sequence set forth in SEQ ID NO: 5 or SEQ ID NO: 6 or SEQ ID NO: 7 or SEQ ID NO: 8.

22. The method according to claim 15, wherein the amplification comprises:
melting the library to give an initial template comprising the forward strand and the reverse strand;
providing a plurality of forward amplification primers immobilized to the surface at 5' ends thereof, wherein the forward amplification primer is capable of hybridizing with a 3' end of the forward strand;
providing a plurality of free reverse amplification primers, wherein the reverse amplification primer is capable of hybridizing with a 3' end of the reverse strand;
hybridizing at least a part of the forward strand with the forward amplification primer to synthesize a nascent strand complementary to the forward strand by extending the forward amplification primer;
removing the forward strand;
hybridizing at least a part of the reverse primer with the nascent strand to synthesize a complementary strand of the nascent strand by extending the reverse amplification primer; and
performing template-walking amplification by using the nascent strand or the complementary strand of the nascent strand as a template and the reverse amplification primer or the forward amplification primer as a primer to give a solid substrate having a surface with a plurality of first single-stranded nucleic acids immobilized thereon.

23. The method according to any one of claims 14 to 22, wherein constructing the library comprises:
providing a double-stranded insert;
ligating adapters to the two ends of the insert to give an adapter - insert - adapter double-stranded nucleic acid molecule, wherein the adapters are double-stranded nucleic acid molecules with predetermined sequences, the adapters consist of a first strand and a second strand that are partially complementary, and a 3' end of the first strand comprises a modification;
providing a first amplification primer and a second amplification primer, wherein a 3' end of the first amplification primer is capable of hybridizing with the 3' end of the first strand of a non-complementary part, a 3' end of the second amplification primer is capable of hybridizing with a 3' end of a complementary strand of the second strand of the non-complementary part, and the second amplification primer comprises the first site and the tag;
synthesizing a new strand complementary to the adapter - insert - adapter using the first amplification primer;
synthesizing a complementary strand of the new strand using the second amplification primer to give a library template comprising the tag and the first site; and
amplifying the library template using the first amplification primer and the second amplification primer to give the library, wherein a forward strand of the library comprises the first strand.

24. The method according to any one of claims 14 to 22, wherein constructing the library comprises:
providing a double-stranded insert;
ligating adapters to the two ends of the insert to give an adapter - insert - adapter double-stranded nucleic acid molecule, wherein the adapters are double-stranded nucleic acid molecules with predetermined sequences, the adapters consist of a first strand and a second strand that are partially complementary, the second strand of a non-complementary part comprises the tag and the first site, and a 3' end of the first strand comprises a modification;
providing a first amplification primer and a second amplification primer, wherein the first amplification primer is capable of hybridizing with the 3' end of the first strand of a non-complementary part, and the second amplification primer is capable of hybridizing with a 3' end of a complementary strand of the second strand of the non-complementary part; and
amplifying the adapter - insert - adapter using the first amplification primer and the second amplification primer to give the library, wherein a forward strand of the library comprises the first strand.

25. The method according to claim 23 or 24, wherein the modification is selected from at least one of an amino modification, a dideoxynucleotide modification, and a PEG modification.

26. The method according to claim 23, wherein the adapter comprises a sequence set forth in SEQ ID NO: 9 and SEQ ID NO: 10.

27. The method according to claim 26, wherein the first amplification primer and the second amplification primer comprise sequences set forth in SEQ ID NO: 11 and SEQ ID NO: 12 or sequences set forth in SEQ ID NO: 11 and SEQ ID NO: 13, respectively.

28. A kit for implementing the method according to any one of claims 1 to 28, comprising the solid substrate and the first sequencing primer.

29. A system for implementing the method according to any one of claims 1 to 28, comprising a memory for storing data comprising a computer-executable program, and a controller for executing the computer-executable program, wherein executing the computer-executable program comprises performing the method according to any one of claims 1 to 28.
